# EUROPEAN PATENT APPLICATION

(11) **EP 1 568 784 A2**
(43) Date of publication of application: **31.08.2005**
(21) Application number: 05001838.1
(22) Date of filing: 28.01.2005
(51) Int. Cl.: C12Q 1/68

(54) **Method for monitoring the effect of compounds on Foxc2 expression**

(30) Priority: 27.02.2004 SE 0400489
(71) Applicant: LeanGene AB, 413 45 Göteborg (SE)
(72) Inventor: Enerbäck, Sven, 431 63 Mölndal (SE); Cederberg, Anna, 413 09 Göteborg (SE)
(74) Representative: Becker Kurig Straus

(57) **Abstract**

The present invention relates to a method for monitoring/detecting compounds capable to regulate the transcription and/or expression of the forkhead transcription factor Foxc2, which compounds may be useful for the treatment of obesity related diseases and diabetes. The screening for the compounds has been performed in recombinant mouse cells containing the Foxc2 gene and the β-galactosidase gene in a suitable targeting vector.

## Description

### Field of the invention

The present invention relates to a method for monitoring/detecting the effect of test compounds on the regulation of the forkhead transcription factor Foxc2, which compounds may be useful for the treatment of various diseases, such as obesity, type 2 diabetes, insulin resistance and diet induced insulin resistance. The screening for the compounds has been performed in recombinant mouse cells containing the Foxc2 gene and the β-galactosidase gene in a suitable targeting vector.

### State of the art

Obesity and related diseases are an increasing problem within the population and require a change in life-style with interventions on the individual behaviour (for example diet and exercise). For many individuals this will not be sufficient and a combination of life-style changes with anti-obesity drugs may be essential. The understanding of the molecular basis for metabolic regulation in insulin responsive tissues (e.g. adipose tissue and skeletal muscle) will help in the development of new analytic tools for defining future drug-based intervention strategies of obesity and type 2 diabetes. Numerous studies have demonstrated a relationship between obesity and insulin resistance, and recent epidemiologic studies have linked the increasing prevalence of diabetes to the obesity epidemic (see Harris et al., The Third National Health and Nutrition Examination Survey, 1988-1994. Diabetes Care 21, 518-524 (1998)).

Diabetes related diseases are caused by multiple factors and may be characterized by elevated levels of plasma glucose (hyperglycemia). In general, there are two recognized forms of diabetes. Type 1 diabetes, or insulin-dependent diabetes mellitus (IDDM), in which patients produce small amounts or no insulin, and type 2 diabetes, the so-called noninsulin-dependent diabetes mellitus(NIDDM), in which the patients produce insulin and even exhibit plasma levels of insulin, which are similar or higher than in comparison with non-diabetic subjects. Type 1 diabetes may be treated by the administration of insulin in high amounts. Patients with type 2 diabetes develop quite often the so-called "insulin resistance", characterized by the effect that the stimulation of insulin on the glucose and lipid metabolism is diminished. It may be assumed that insulin resistance occurs primarily due to a receptor binding defect and results inter alia in an insufficient activation of the glucose uptake. Patients with type 2 diabetes exhibit at increased risk of developing different cardiovascular complications.

Diabetes may be treated by the administration of a variety of therapeutic compounds including different insulin sensitizers. The plasma level of insulin is increased by the administration of sulfonylureas or meglitinides, which stimulate the insulin secretion, and/or by injection of insulin when the above compounds get ineffective, may result in insulin concentrations high enough to stimulate insulin-resistant tissues. However, dangerously low levels of plasma glucose can result, and increasing insulin resistance due to the even higher plasma insulin levels may occur.

Other possibilities for the treatment of obesity and diabetes related diseases pertain in the identification of metabolic regulators, for example to the forkhead/winged helix transcription factors. This family of proteins has been shown to play a role in embryonic pattern formation, regulation of tissue-specific gene expression, and tumorigenesis (see Carlsson et al.; Dev Biol 250, 1-23 (2002) and Kume et al.; Genes Dev 15, 2470-2482 (2001)). One member of this protein family, Foxc2 promoted greater sensitivity of the protein kinase A (PKA)-signalling pathway, owing to altered subunit composition of PKA, as well as increased levels of β-adrenergic receptors. As a result of these changes in gene expression, Foxc2 decreased total body lipid content and levels of free fatty acids (FFA); and increased insulin sensitivity as well as thermogenesis in white adipose tissue. The expression in mice resulted in an animal protected from diet-induced obesity and insulin resistance.

The Foxc2 gene has already been used for example in the US 6,709,860, which pertains to transgenic non-human mammalian animals capable to express the human FKHL14/Foxc2 gene in their adipose tissue. In addition, methods for identifying compounds useful for the treatment of medical conditions related to obesity or diabetes are disclosed. The compounds are capable to stimulate the expression of the human FKHL14/Foxc2 gene, or the biological activity of a polypeptide encoded by the human FKHL14/Foxc2 gene and may be used for the treatment of medical conditions related to malnutrition.

Isolated promoter regions of the mammalian transcription factor Foxc2 are known from the WO 0227008. Said document also relates to screening for agents, which are capable to modulate the expression of Foxc2 and which have a potential use for the treatment of medical conditions related to obesity.

From the WO 03064467 complexes of the Foxc2 protein with other proteins are known, which may be used likewise for the identification of agents. Said agents may be used for the treatment of medical conditions such as obesity, hypertriglyceridemia, diet-induced insulin resistance, and/or diabetes.

The prior art merely relates to possibilities for the identification of compounds which may alter the Foxc2 expression. Nevertheless, the construction of suitable assays for the identification of said compounds is not disclosed.

### Object of the invention

A problem of the present invention is, therefore, the provision of a suitable assay system capable to monitor/detect test compounds, which may alter the Foxc2 transcription and/or expression.

The present invention solves the above-mentioned problem by the provision of an assay system as set forth in claim 1, which provides a fast and reliable method for the identification of test compounds altering the Foxc2 expression, which method may be performed in a high throughput manner.

### Figures

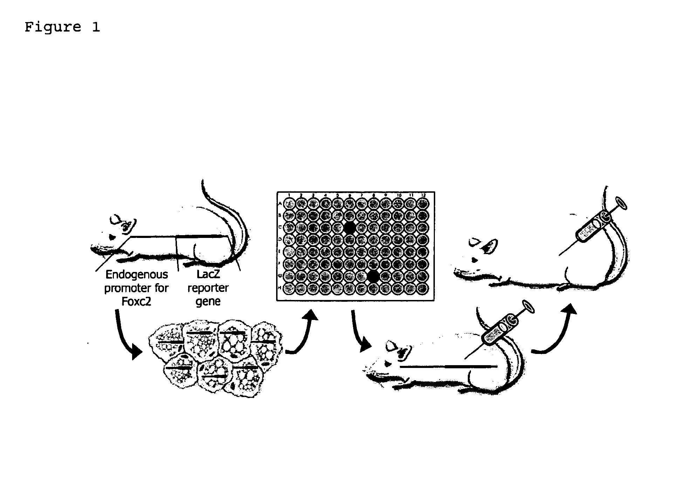

Figure 1 discloses a concept for the usage of LacZ knock-in mice to screen for test compounds.

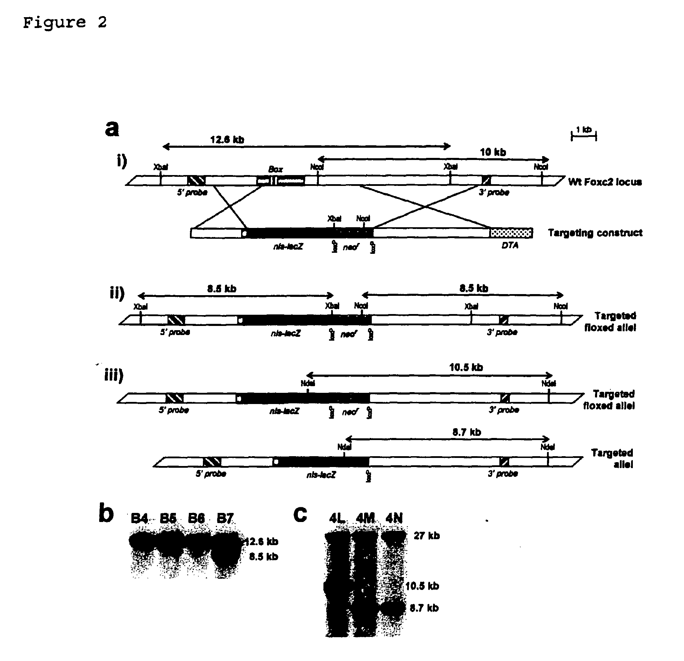

Figure 2 shows the generation of Foxc2nLacZ knock-in mice. (a) represents a schematic representation of the relevant part of the mouse Foxc2 locus, the targeting strategy and targeting construct. The targeting vector was generated by inserting the selection cassette, containing the β-galactosidase gene (LacZ) with a nuclear localization signal (nls-lacZ) and a floxed (flanked by loxP sites) neomycin resistance gene (neo^{r}) 17 amino acids down-stream of the start codon (ATG) of the Foxc2 gene. In the resulting construct and in the mutant allele the LacZ gene is thereby fused in frame to the beginning of the Foxc2 coding sequence, which results in expression of a Foxc2nLacZ fusion protein under the control of the endogenous Foxc2 promoter. (a-i) and (a-ii) show the result of a correct targeting event by homologues recombination. (a-iii) shows how the floxed neo^{r} gene is removed by recombination in correct targeted ES cell clones; this is done by transfection with a PGK-cre expression plasmid. As a final result this construct deleted 3.7 kb of endogenous Foxc2 sequence, including sequences encoding the DNA-binding winged helix domain. (b) shows a southern blot analysis of Foxc2 disruption. The 5' probe was used to detect a 12.6 kb fragment in the wild-type allele and a 8.5 kb fragment in the mutant allele following XbaI digestion of genomic DNA isolated from ES cells. Homologous recombination was detected in 0.4% (4/960) of the ES cell clones screened. In (c) southern blot analysis is used to verify the correct removal of neo^{r} gene. The 3' external probe was used to detect a 10.5 kb fragment in the originally targeted floxed neo^{r} allele and an 8.7 kb fragment in the correct targeted allele with removed neo^{r} gene following NdeI digestion of genomic DNA isolated from ES cells.

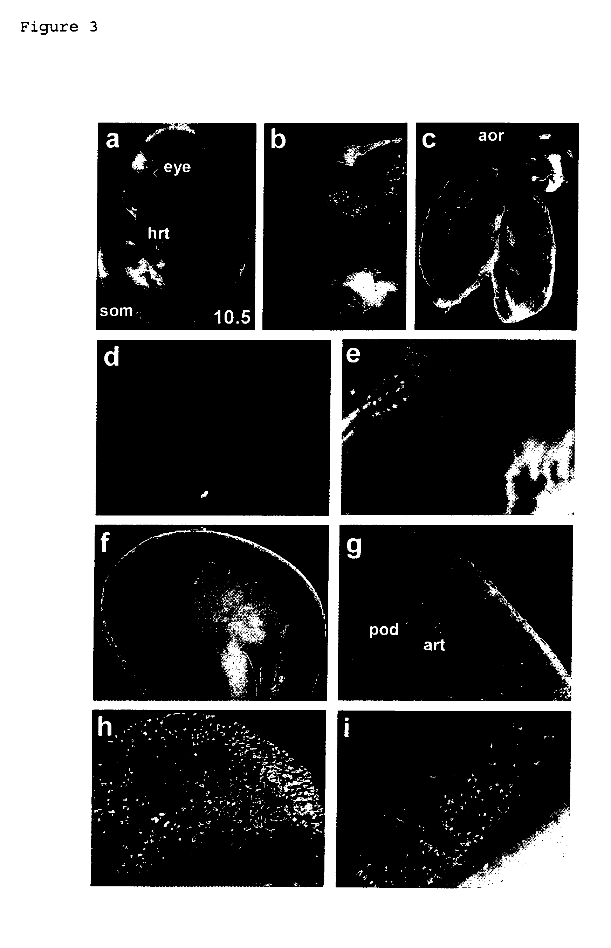

Figure 3 shows the expression pattern of Foxc2 in E10.5 embryo and adult tissues, wherein an X-gal staining was performed overnight. In (a) the whole-mount X-gal staining of Foxc2nLacZ +/- E10.5 embryos is shown. Foxc2 expression may be seen in e.g. somites (som), developing heart (hrt), and periocular mesenchyme around the developing eye (eye). (b)-(i) relate to the X-gal staining on different dissected tissue pieces from six week old Foxc2nLacZ +/- mice. Wt littermates were stained in parallel as control (not shown). (b) refers to the expression of Foxc2 in the brain, wherein it is for example expressed in meninges. (c) shows the expression of Foxc2 in the adult aortic arch. (d) lympahtic vessels in myocardium, but not in the cardiomyocytes, as well as in oviduct (e) show Foxc2 expression. In (f) Glomeruli in the kidney cortex stain for β-galactosidase activity. Due to diffusion effects only glomeruli in the cortex are stained. (g) is with greater magnification and shows the expression of Foxc2 in arterioles (art), supplying glomeruli with blood. The cells in the glomeruli demonstrating Foxc2 expression are podocytes (pod). In (h) adult hair follicles are shown as another site for Foxc2 expression. In (i) the Foxc2 expression in white abdominal adipose tissue is shown.

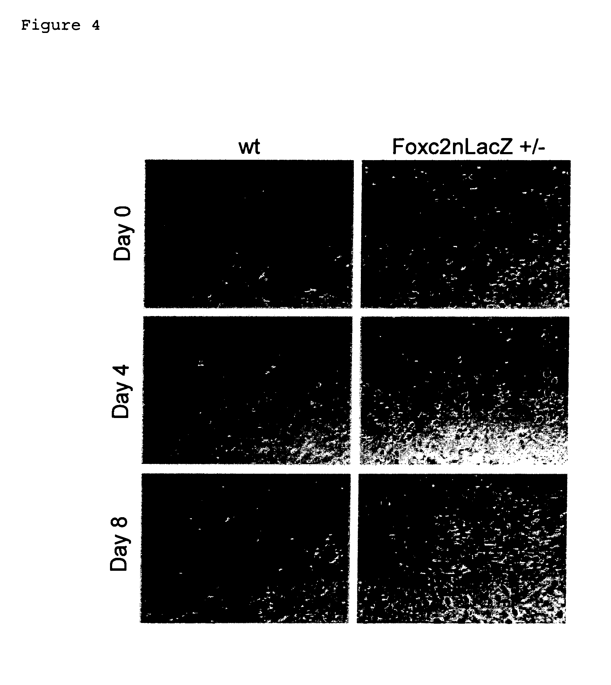

Figure 4 specifies the expression of Foxc2 during adipocyte differentiation of mouse embryonic fibroblasts (MEFs), which were isolated from Foxc2nLacZ +/- E13.5 embryos as well as wt littermates as control. The MEFs were treated with adipocyte differentiation mix (0.5 mM IBMX, 0.25 µM Dex and 10 µg/mL insulin) and X-gal stained at different time points after start of differentiation. Expression of Foxc2 increases during the course of adipocyte differentiation as judge by increasing β-galactosidase activity.

### Detailed description of the present invention

The present invention provides a method for monitoring and/or detecting the regulation of a gene construct with a test compound. Said gene construct comprises the gene encoding the forkhead transcription factor Foxc2 or parts thereof, and a reporter gene, such as a gene encoding e.g. β-galactosidase, GFP, or luciferase, or other suitable genes allowing detection, such that an in frame fusion or an operative linkage is provided between these components (the gene encoding the forkhead transcription factor Foxc2 or parts thereof, and the reporter gene) to report the transcription/expression of the construct, if the Foxc2 promoter is influenced by a chemical substance.

As the gene encoding the forkhead transcription factor Foxc2 or parts thereof any nucleic acid sequence is envisaged that comprises the promotor region of the forkhead transcription factor Foxc2 and either the enitre gene sequence or parts thereof, in particular those parts ensuing the promotor region, i.e.the 5'-region of the gene.

The method according to the present invention is characterized in that (a) said gene construct is inserted in a vector, which is preferably a targeting vector, and subsequently introduced in mouse primary cells or mouse embryonic fibroblasts, which shall be heterozygous for said gene construct and which process is also referred herein as "knock-in". Therefore, the respective Foxc2 fusion gene with lacZ has been created in such a way that the LacZ gene is under transcriptional control of an endogenous Foxc2 promoter. After insertion of said construct in a suitable targeting vector, the vector is introduced into mouse cells.

In step (c) a cell line from said primary cells or embryonic fibroblasts is established, followed by an optional differentiation step of said cells into tissue cells. This step may be effected by any of the methods for differentition known to the skilled person. Subsequently, in step (e) the cells are exposed to a substance of interest (the test compound), which may, if desired, be carried out during an optional incubation step, which facilitates uptake and transport of the test substance in and within the cell. After a given time period, which will be selected by the skilled eprson depending on the assay to be run, the transcription and/or expression level of the reporter gene may be determined by using suitable means, such as e.g. Northern blot, PCR, Wetser-blots and dye and/or fluorescence detection of the reporter gene product. The compound yileding a desired result, i.e. activation or increase or decrease of the transcription or expresson of the reporter gene may be selected for further investigation.

The gene construct as used herein refers to any combination of a particular gene with other nucleic acid sequences by for example ligation techniques. Said gene construct may be obtained by any recombinant technique known to the skilled person and outlined for example in Maniatis et al.; Molecular Cloning: A Laboratory Manual 2nd ed., (1989). The same applies likewise to respective techniques for introducing a gene construct in a targeting vector and the subsequent transfection of the host cells. The transfection techniques may comprise preferably electroporation.

The method according to the present invention may be surprsingly also carried out in small sample volumes, which allow the set-up of high troughput screening systems. Such a system employs for example micro-titer plates of any size having an optional coating of the wells. Micro-titer plates used for this purpose may comprise preferably 96 well micro-titer plates with gelatine coating.

The monitoring/detecting of the regulation of said gene construct is performed via a "reporter means", which may be the product of the reporter gene, either on the transcriptional or the expression level, i.e. for example via enzymatic activity (for example by X-gal staining in case of using the lacZ gene as the reporter gene or when using luciferase applying the respective substrate) or via a protein (e.g. GFP that emits fluorescence when irradiated), or via northern blotting, realtime RT-PCR, western blotting etc.. The β-galactosidase enzyme is an conventioanl and easy means, that catalyzes the hydrolysis of X-gal (5-bromo-3-indoyl-β-D-galactopyranoside), with the effect that cells containing a β-galactosidase gene will appear blue after incubation with X-gal. The blue cells may easily be visualized by eye or microscope. As the β-gal activity (the protein product of the LacZ gene) may be measured easily, the cells may be subjected to in vitro high through-put screens using compound libraries. Likewise to X-gal staining, western blotting is directed to the detection of proteins, whereas northern blotting and RT-PCR are methods for locating/quantifying RNA. These methods are all well known to the skilled person.

The gene construct used for the method according to the present invention is preferably designed such that the start codon of the reporter gene, e.g. the lacZ gene, is inserted 17 amino acids downstream of the Foxc2 start codon, which enables the transcription and expression of a reporter gene product still exhibiting its native properties, while being under the control of the Foxc2 promotor. Hence modulation of the Foxc2 transcription/expression with test compounds may easily be tracked by monitoring the transcritpion and/or expression of the reporter gene/reporter gene product. Insulin, which is known to by an inducer of Foxc2 may be utilized as a control.

The construct containing the Foxc2 gene (or parts thereof) with the reporter gene may contain at the respective ligation site between the two components, a nuclear localization signal (nls) to ensure a distinct nuclear detection of reporter gene positive cells, for a reliable report of Foxc2 expression levels, since transcription start, cis-elements and exon/intron structure will be very similar to that of the wild type (wt) Foxc2 allele.

The targeting vector is preferably the pPGKneobpAlox2PGKDTA vector.

The test compound identified in the method according to the present invention is preferably capable to activate and/or increase and/or decrease Foxc2 expression by inducing/repressing the Foxc2 promoter and thereby, initiating the expression of the reporter gene. After further investigations such a test compound may be suitable to be used as a lead compound in clinical studies. I.e. not only a positive induction is envisaged in the present invention, since potential inhibitors of the Foxc2 expression also have suitable applications.

The capability of said test compound to activate Foxc2 expression is preferably verified in mouse models, which may comprise Foxc2:nls:β-galactosidase as already mentioned-above or other mouse models, such as knock-in mice or diabetic and obese mouse models, which are known to the skilled person.

The test compound obtained by the method according to the present invention is preferably capable to induce adaptive thermogenesis, insulin sensitivity and an increased sensitivity of the protein kinase A signaling pathway, thereby indicating inter alia the possible use of said compound to create heat in the sense of consuming stored energies in the form of for example fat.

The test compound obtained by the method according to the present invention is pereferably used for the preparation of a composition for the prevention or treatment of any disease associated with Foxc2 expression levels, which may be for example selected from obesity, type 2 diabetes, insulin resistance, diet induced insulin resistance, hypertriglyceridemia, cancer and an increased plasma levels of free fatty acids.

The following examples illustrate the inventino without limiting it thereto.

### Examples

### Targeting construct

Foxc2 clones were isolated from a mouse 129/SvJ genomic library (Stratagene) using a specific Foxc2 probe. The correct identity of the clones was confirmed by sequencing. To construct the targeting vector, two fragments of the genomic DNA flanking the DNA-binding region were subcloned at convenient restriction sites into the pPGKneobpAlox2PGKDTA vector. The targeting vector contained 2.7 and 5.0 kb of homologous genomic DNA on either side of a floxed neomycin resistance cassette. The 2.7 kb short arm was fused in frame with an nls-LacZ cassette obtained from the pCH110-nls plasmid leaving the first 17 amino acids of Foxc2 open reading frame intact. This construct deleted 3.7 kb, including sequences encoding the DNA-binding winged helix domain, of endogenous Foxc2 genomic sequence.

### ES cells and Foxc2nLacZ mutant mice

R1 ES cells (2x10⁷) were electroporated in Dulbecco's modified Eagle medium (DMEM) containing linearized targeting construct (30 µg). Selection was performed on neomycin resistant mouse embryonic fibroblasts with G418 (300 µg/ml) and ganciclovir (2 µM). Double-resistant colonies were screened by Southern blot analysis. DNA samples were digested with XbaI and probed with a 5' external probe or with NcoI and probed with a 3' external probe. Homologous recombination was detected in 0.4% (4/960) of the ES cell clones screened. The ES clones were subsequently subjected to transfection with a cre expressing plasmid, PGK-cre, to recombinate out the loxP site flanked neomycin resistance cassette. Correct recombination was screened for by Southern blot analysis. DNA samples were digested with NdeI and probed with the 3' external probe. Four correctly targeted ES cell clones were injected into C57B16/J host blastocysts to generate chimeric animals. Male chimeras were bred with C57B16/J females and two of the cell lines were found to generate germ-line transmission, determined by Southern-blot analysis after XbaI digestion. Heterozygous mice were mated on to C57B16/J background for at least five generations. Subsequent progeny and embryos were genotyped by PCR. The PCR primers were designed to use a common 5' primer located upstream of the ATG (5'- GTCCCTTCCTGCTCTCCTG - 3'), and specific primers for the LacZ gene (5'- TCAGTTCGAGGTGCTGTT -3') and the Foxc2 ORF (5'- GCCCGGTAGTAGTTTTGCTC -3'). These primers generate products of 221 bp specific for the mutant allele and 168 bp for the wild-type.

### Generation of Foxc2nLacZ knock-in mice

The strategy of the generation of Foxc2nLacZ knock-in mice is shown in Fig. 2a. A targeting vector was built with a LacZ reporter gene (β-galactosidase) fused in-frame with the Foxc2 gene, 17 amino acids downstream of the start codon (ATG) of Foxc2, to report the Foxc2 expression. To ensure a distinct nuclear staining of β-galactosidase positive cells, a nuclear localization signal (nls) was fused in the ligation point between Foxc2 and LacZ coding sequences. Thereby, a Foxc2-nls-LacZ (Foxc2nLacZ) fusion protein was created with the potential to faithfully report of Foxc2 expression levels since transcription start, cis-elements and exon/intron structure will be very similar to that of the wild type (wt) Foxc2 allele. The targeting vector was electroporated into R1 embryonic stem (ES) cells and clones that had undergone correct homologous recombination was identified by Southern blot analysis (Fig. 2b). In the next step the selection marker (i.e. the neomycin resistance (neo^{r}) gene flanked by loxP sites) was removed by transfection of the identified ES cell clones with a cre-recombinase expressing vector, and correct recombination was monitored with Southern blot analysis (Fig. 2c). Correctly targeted ES cell clones were used to generate chimeric mice. Backcrosses with wt C57BL6/J mice resulted in heterozygous Foxc2nLacZ +/- mice, genotypes of the knock-in mice were verified by Southern blotting or PCR (Fig. 2d). Previously, two other groups have generated mice with targeted inactivation of the Foxc2 gene. Homozygous null animals died embryonically after day E12.5 with a few surviving to birth and dying shortly thereafter, while heterozygous mice have a normal lifespan. Accordingly, heterozygous Foxc2nLacZ+/- mice were viable and fertile but our homozygous Foxc2nLacZ-/- mice as expected died perinatally, demonstrating similar defects as reported previously (data not shown). All comparative studies were carried out on the wild type (wt) +/+ siblings of Foxc2nLacZ+/- knock-in mice.

### MEFs and primary tissue cultures

MEFs were isolated from E13.5 embryos from Foxc2nLacZ +/- heterozygous intercrosses and genotyped by PCR on DNA from the tail. The head, limbs and viscera were removed from embryos and the remains were minced with a razor blade and incubated in 0.25% trypsin, 0.1% EDTA for 1 h at 37°C. The cell suspension was plated in DMEM containing 10% fetal calf serum and antibiotics (100 units of penicillin and 100 µg/mL streptomycin). Once the cultures reached confluency, 1x10⁶ cells were plated on 10-cm tissue culture plates (Falcon) and considered passage 0.

### Adipocyte differentiation of MEFs

Primary MEFs from both wild type and Foxc2nLacZ knock-in mice were maintained in DMEM:*****F-12 nutrient***** mix (DMEM:F12, Gibco BRL). Medium was supplied with 10% fetal bovine serum (FBS, Gibco BRL) and antibiotics. Confluent plates of cells were induced to differentiate with BME or DMEM:F12 containing 10% FBS. For adipocyte differentiation in MEF four different protocols were tested. Protocol #1: culture medium supplemented with 0.5 mM IBMX, 0.25 µM Dex and 10 µg/mL insulin for the first 3 days. Subsequently, cultured cells were maintained in standard medium supplemented with 5 µg/mL insulin only. Protocol #2: standard medium was supplemented with 125 µM indomethacine and 5 µg/mL insulin. After the first 3 days, medium was supplemented with 5 µg/mL insulin only. Protocol #3: standard medium including 2 nM triiodothyronine (T₃) and 17 nM insulin throughout the differentiation period. Protocol #4: standard culture medium, containing 82 µg/mL ascorbic acid and 10 mM β-glycerophosphate throughout the differentiation period. Acute insulin stimulation with 200nM insulin was preceded by basaling in serum free DMEM for 1 hour.
For the X-gal staining, the cells were fixed with 1% glutaraldehyde, and β-galactosidase (β-gal) enzyme activity was detected using a commercially available kit: beta-GLO, Promega,USA, according to the manufacturer's recommendations.

### Expression of LacZ reporter from the Foxc2 locus

To verify the functionality of our Foxc2nLacZ reporter mice the expression of Foxc2nLacZ fusion protein was monitored by X-gal staining of whole-mount embryos and tissue pieces from adult mice, and compared with the known expression pattern for Foxc2. The Foxc2 expression pattern during embryonic development have been scrutinized fairly well, previously reported expression studies of Foxc2 using in situ hybridization demonstrated that Foxc2 is transcribed early in embryogenesis in several developing tissues such as heart, blood vessels, nasal septum, kidney, periocular mesenchyme and condensing cartilage that forms bone. Adult expression of Foxc2 has been studied much less extensive, being assessed mostly by Northern blots and realtime RT-PCR assays, in which Foxc2 expression has been demonstrated in adipose tissue and skeletal muscle. Theresults with X-gal staining of Foxc2nLacZ embryos and tissue pieces demonstrated a specific expression pattern. The use of whole-mounts, sectioning and X-gal staining on tissues from Foxc2nLacZ reporter mice is a very powerful tool for dissecting intricate Foxc2 expression patterns. At embryonic stage E10.5 days post-coitum (dpc) β-galactosidase activity is detected in among others presomitic mesoderm, somites, mesenchymal cells surrounding the eye (periocular mesenchyme) and the developing heart (Fig. 3a), corresponding well to earlier studies with whole-mount in situ hybridization against Foxc2 mRNA. It is also clear that one site of expression of Foxc2 in the adult brain is the meninges (Fig. 3b). Foxc2 is known to be important for correct lay out and remodelling of the aortic arch during embryonic development and even in adult mice there is a very prominent expression of Foxc2 in the aortic arch (Fig. 3c). The same type of staining pattern is observed in adult oviduct (Fig. 3e) indicating expression of Foxc2 in the lining of some kind of vessel. Foxc2 expression is also dected in adult kidney (Fig. 3f), especially in the podocytes (Fig. 3g). Podocytes are highly specialized cells found in the vertebrate and invertebrate kidney and make up a major portion of the filtration barrier between blood and urinary spaces. Expression of Foxc2 has previously been demonstrated in this cell type in late gestation embryos as well as a day 1 neonate and adult as verified by co-expression of the podocyte-specific marker nephrin. Moreover, arterioles, supplying the glomerulus with blood, also expressed Foxc2 (Fig. 3g). A totally new site of observed Foxc2 expression is hair follicles (Fig. 3h). And finally, in accordance with previous Northern and Western blots, white adipose tissue from the abdomen demonstrated β-galactosidase activity (Fig. 3i).

### Monitoring Foxc2 expression in MEFs

A cell based high-throughput screening system has been established by isolation of MEFs from heterozygous Foxc2nLacZ +/- E13.5 embryos as well as from wt embryos as a control. As detected by increased X-gal staining, Foxc2nLacZ protein levels increased during differentiation and moreover levels could be induced by acute treatment with insulin and TNFα (Fig. 4).

### X-gal staining of whole-mount embryos and tissue pieces

Whole-mount embryos and tissue pieces were dissected in phosphate-buffered saline (PBS) and fixed in 4% paraformaldehyd (PFA) at 4°C in dark for 1 hour or overnight. Rinsing was performed for 3x 15 minutes with rinse buffer (5 mM EGTA, 0.01% Deoxycholate, 0.02% NP40, 2 mM MgCl₂ in PBS), and followed by incubation in staining solution [5 mM EGTA, 0.01% Deoxycholate, 0.02% NP40, 2 mM MgCl₂, 5 mM K₃Fe(CN)₆, 5 mM K₄Fe(CN)₆, and 1 mg of X-gal/ml in PBS] overnight at 37°C to detect ß-galactosidase activity. β-galactosidase expressing tissues were located and photographed under a dissecting microscope.

## Claims

1. Method for monitoring/detecting the regulation of a gene construct, comprising an in frame fusion of the forkhead transcription factor Foxc2 with a reporter gene, with a test compound comprising the steps of:
(a) inserting said gene construct in a targeting vector;
(b) transfecting mouse primary cells or mouse embryonic fibroblasts with said vector;
(c) establishing a cell line from said primary cells or fibroblasts;
(d) optionally differentiating said cells into tissue cells;
(e) treating the cells from step (c) or (d) with said test compound;
(f) optionally allowing incubation;
(g) detecting the change in transcription and/or expression of said reporter gene; and
(h) optionally selecting the test compound which alters the regulation of the forkhead transcription factor Foxc2;
wherein said cells are heterozygous for said gene construct.

2. The method according to claim 1, which is carried out in a miniaturised high-throughput format.

3. The method according to any of the preceding claims, wherein said tissue cells from step (d) are selected from muscle cells, liver cells, or preferably from adipocytes.

4. The method according to any of the preceding claims, wherein monitoring/detecting the regulation of said gene construct is performed by detecting the product of the reporter gene, northern blotting, realtime RT-PCR or western blotting.

5. The method according to any of the preceding claims, wherein the Foxc2:reporter gene- gene construct is obtained by inserting the start codon of the reporter gene 17 amino acids downstream of the Foxc2 start codon.

6. The method according to any of the preceding claims, wherein in the ligation point of said Foxc2:reporter gene construct a nls has been inserted.

7. The method according to any of the preceding claims, wherein the targeting vector is the pPGKneobpAlox2PGKDTA vector.

8. The method according to any of the preceding claims, wherein said test compound is capable to activate or repress Foxc2 expression.

9. The method according to any of the preceding claims, wherein the capability of said test compound to modulate Foxc2 expression is verified in mouse models, wherein said mouse models comprise Foxc2:nls:β-galactosidase knock-in mice or diabetic and obese mouse models.

10. The method according to any of the preceding claims, wherein said test compound is capable to induce adaptive thermogenesis, insulin sensitivity and an increased sensitivity of the protein kinase A signalling pathway.

11. Use of the test compound according to any of the preceding claims for the preparation of a composition for the prevention or treatment of a disease associated with Foxc2 expression levels.

12. The use according to claim 11, wherein said disease associated with Foxc2 expression levels is selected from obesity, type 2 diabetes, insulin resistance, diet induced insulin resistance, hypertriglyceridemia, cancer and increased plasma levels of free fatty acids.
